(19) Europäisches Patentamt
European Patent Office
Office européen des brevets

(11) Veröffentlichungsnummer: **0 100 516**
**B1**

(12) # EUROPÄISCHE PATENTSCHRIFT

(45) Veröffentlichungstag der Patentschrift: **14.10.87**

(51) Int. Cl.⁴: **C 07 C 69/757**, A 61 K 31/225, C 07 C 67/08

(21) Anmeldenummer: **83107337.4**

(22) Anmeldetag: **26.07.83**

(54) **3-Beta-(3'-(Carboxypropionyloxy))-ursa-9(11),12-dien-28-carbonsäure sowie ihre Salze, Verfahren zu ihrer Herstellung und diese Verbindungen enthaltende Arzneimittel.**

(30) Priorität: **26.07.82 IT 2256682**

(43) Veröffentlichungstag der Anmeldung: **15.02.84 Patentblatt 84/7**

(45) Bekanntmachung des Hinweises auf die Patenterteilung: **14.10.87 Patentblatt 87/42**

(84) Benannte Vertragsstaaten: **AT BE CH DE FR GB IT LI LU NL SE**

(56) Entgegenhaltungen:
**DE-A-2 412 355**
**DE-B-1 076 684**
**FR-A-6 906**
**GB-A-1 475 075**

(73) Patentinhaber: **I.S.F. Società per Azioni, Via Leonardo da Vinci, 1, I-20090 Trezzano s/N Milano (IT)**

(72) Erfinder: **Clavenna, Geatano, Dr., Via Ospiate 22, Rho (Milano) (IT)**
Erfinder: **Farina, Carlo, Via Statale 105, Valsolda (Como) (IT)**
Erfinder: **Pinza, Mario, V. per Cesano Boscone 32, Corsico (Milano) (IT)**
Erfinder: ***Pifferi, Giorgio, v. Indipendenza 20,* Milano (IT)**

(74) Vertreter: **Beszédes, Stephan G. Dr., Münchener Strasse 80a Postfach 1168, D-8060 Dachau (DE)**

**EP 0 100 516 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die Erfindung betrifft die neuen Verbindungen 3β-[3'-(Carboxypropionyloxy)]-ursa-9(11),12-dien-28-carbonsäure und ihre Salze, ein Verfahren zu ihrer Herstellung und diese Verbindungen enthaltende Arzneimittel, insbesondere solche mit geschwürhemmender Wirkung beziehungsweise Antiulkuswirkung und viricider Wirkung beziehungsweise Antiviruswirkung.

Es sind bereits Verbindungen mit polycyclischer Struktur und Wirksamkeit gegen geschwürbildende Entzündungen des Verdauungssystemes bekannt. Unter diesen ist ein Derivat der Glyzyrrhetinsäure, nämlich Glyzyrrhetinsäurehydrogensuccinat {3β-[3'-(Carboxypropionyloxy)]-11-oxo-olean-12-en-30-carbon-säure} <Carbenoxolon>, am interessantesten. 3β-[3'-(Carboxy-propionyloxy)]-11-oxo-olean-12-en-30-carbonsäure <Carbenoxolon> hat aber außer der ausgeprägten geschwürhemmenden Wirksamkeit auch eine deutliche Mineralcorticoidwirksamkeit, welche sich in einer erhöhten Natrium- und Wasserretention und einer erhöhten Kaliumausscheidung äußert. Diese unerwünschte Mineralcorticoidwirksamkeit macht die Verwendung der 3β-[3'-(Carboxypropionyloxy)]-11-oxo-olean-12-en-30-carbonsäure <des Carbenoxolones> nicht völlig zufriedenstellend in der Therapie, zumindest nicht in denjenigen Fällen, in welchen eine Niereninsuffizienz und/oder Bluthochdruck vorliegt beziehungsweise vorliegen. Ferner ist es bekannt, daß die gleichzeitige Verabreichung eines kaliumschonenden Diureticums den Verlust der geschwürhemmenden Wirksamkeit der 3β-[3'-(Carboxypropionyloxy)]-11-oxo-olean-12-en-30-carbonsäure <des Carbenoxolones> selbst herbeiführen kann.

Der Erfindung liegt daher die Aufgabe zugrunde, überlegene pharmakologische Wirkungen aufweisende neue Ursa-12-en-28-carbonsäurederivate, ein Verfahren zur Herstellung derselben und diese Verbindungen enthaltende Arzneimittel zu schaffen.

Das Obige wurde überraschenderweise durch die Erfindung erreicht.

Es wurde nämlich überraschenderweise festgestellt, daß 3β-[3'-(Carboxypropionyloxy)]-ursa-9(11),12-dien-28-carbonsäure und ihre Salze wertvolle pharmakologische, insbesondere geschwürhemmende und viricide, Wirkungen praktisch frei von unerwünschten Nebenwirkungen, insbesondere ohne nennenswerte Mineralcorticoidwirksamkeit, haben.

Gegenstand der Erfindung sind daher 3β-[3'-(Carboxypropionyloxy)]-ursa-9(11),12-dien-28-carbonsäure der Formel

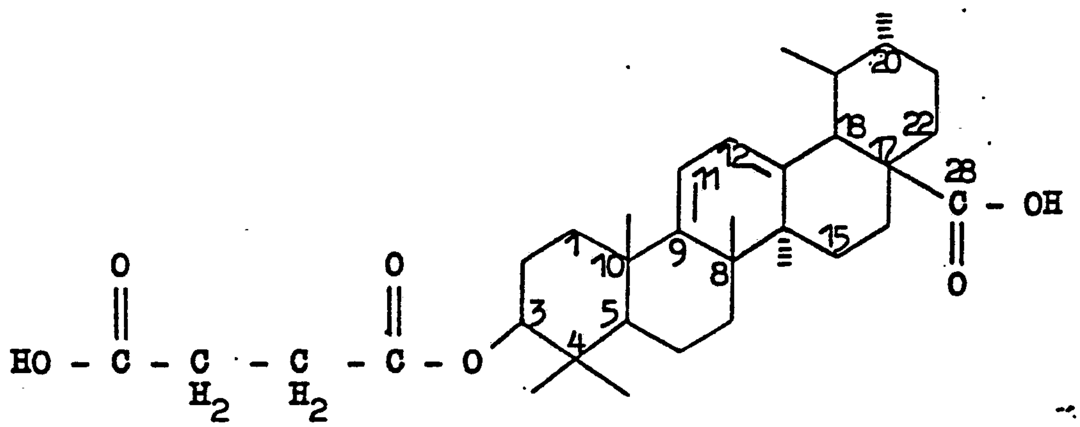

sowie ihre Salze.

Als Salze der erfindungsgemäßen 3β-[3'-(Carboxypropionyloxy)]-ursa-9(11),12-dien-28-carbonsäure kommen die pharmazeutisch brauchbaren in Frage. Bevorzugte Salze der erfindungsgemäßen 3β-[3'-(Carboxypropionyloxy)]-ursa-9(11),12-dien-28-carbonsäure sind solche mit Alkalimetallen, insbesondere Natrium oder Kalium, Erdalkalimetallen, wie Calcium, Ammoniak, Aminen, insbesondere Triäthylamin, 4-Aminobenzoesäure-(2'-diäthylaminoäthyl)-ester [Procain], Dibenzylamin oder N,N'-Dibenzyläthylendiamin, und basischen Aminosäuren, insbesondere Lysin.

Ein besonders bevorzugtes erfindungsgemäßes Salz ist das Dinatriumsalz der 3β-[3'-(Carboxypropionyloxy)]-ursa-9(11),12-dien-28-carbonsäure.

Gegenstand der Erfindung ist auch ein Verfahren zur Herstellung dar erfindungsgemäßen Verbindungen, welches dadurch gekennzeichnet ist, daß in an sich bekannter Weise 3β-[Hydroxy]-ursa-9(11),12-dien-28-carbonsäure succinyliert wird, worauf in an sich bekannter Weise gegebenenfalls die erhaltene ein Halbsuccinat (Hemisuccinat) darstellende 3β-[3'-(Carboxypropionyloxy)]-ursa-9(11),12-dien-28-carbonsäure in Salze überführt wird beziehungsweise gegebenenfalls die erhaltenen Salze der 3β-[3'-(Carboxypropionyloxy)]-ursa-9(11),12-dien-28-carbonsäure in die freie 3β-[3'-(Carboxypropionyloxy)]-ursa-9(11),12-dien-28-carbonsäure oder in andere Salze überführt werden.

Ferner sind erfindungsgemäß Arzneimittel, welche 1 oder mehr der erfindungsgemäßen Verbindungen als Wirkstoff beziehungsweise Wiikstoffe, gegebenenfalls zusammen mit 1 oder mehr üblichen pharmazeutischen Konfektionierungsmittel(n), enthalten, vorgesehen.

Die erfindungsgemäßen Verbindungen haben nämlich wie bereits erwähnt wertvolle pharmakologische, insbesondere geschwürhemmende und viricide, Wirkungen.

So haben die erfindungsgemäßen Verbindungen eine hohe Wirksamkeit bei der Behandlung von geschwürbildenden Entzündungen des Verdauungssystemes, da sie eine ausgeprägte zellschützende Wirkung zeigen, wobei sie bei den therapeutisch wirksamen Dosen praktisch frei von jeglicher Mineralcorticoidwirksamkeit sind. Es erwies sich, daß die erfindungsgemäßen Verbindungen im Vergleich zur 3β-[3'-(Carboxypropionyloxy)]-11-oxo-olean-12-en-30-carbonsäure <Carbenoxolon> bei denselben Dosen eine ähnliche geschwürhemmende Wirksamkeit aufweisen, jedoch keine nennenswerte Natriumretention und keine nennenswerte Kaliumausscheidung herbeiführen.

I) Geschwürhemmende Wirksamkeit

Die geschwürhemmende Wirksamkeit des erfindungsgemäßen Dinatriumsalzes der 3β-[3'-(Carboxypropionyloxy)]-ursa-9(11),12-dien-28-carbonsäure wurde unter Verwendung der folgenden 3 Modelle von künstlich erzeugten Magenschäden an Ratten bestimmt wobei als Vergleichssubstanz die anerkannt gut wirksame 3β-[3'-(Carboxypropionyloxy)]-11-oxo-olean-12-en-30-carbonsäure <Carbenoxolon> gleicher Wirkungsrichtung herangezogen wurde: Durch Streß erzeugte Magenschäden, durch Acetylsalicylsäure erzeugte Magenschäden und mit absolutem Äthanol erzeugte Magenschäden.

a) Durch Streß erzeugte Magenschäden

Es wurden 60 Stunden lang nüchtern gehaltene weibliche Sprague-Dawley-Ratten als Versuchstiere verwendet. Die Tiere wurden in passenden beschränkenden Behältern unbeweglich gemacht beziehungsweise immobilisiert und 2 Stunden lang bei 4° C in diesem Zustand gehalten.

Die zu untersuchenden Substanzen wurden in Lösung in destilliertem Wasser in Dosen von 100 mg/kg Körpergewicht in Volumina von 5 ml/kg Körpergewicht 15 Minuten, bevor die Tiere dem Streß ausgesetzt wurden, peroral verabreicht.

Am Ende des Versuches wurde das Auftreten der Magenschäden an der Magenschleimhaut der behandelten Tiere und ihre Schwere auf der Grundlage des aus der Summe der Länge aller Schäden, ausgedrückt in Millimetern, erhaltenen Indexes bei Verwendung von Blindversuchstieren, denen nichts verabreicht wurde, als Kontrolle bestimmt.

b) Mit Acetylsalicylsäure erzeugte Magenschäder

Es wurde 24 Stunden lang nüchtern gehaltete männliche Sprague-Dawley-Ratten verwendet. Die Tiere wurden mit Acetylsalicylsäure in einer peroralen Dosis von 200 mg/kg behandelt.

Die zu untersuchenden Substanzen wurden gleichzeitig mit dem Schädigungsmittel in Lösung in destilliertem Wasser in Dosen von 100 mg/kg Körpergewicht in Volumina von 5 ml/kg Körpergewicht peroral verabreicht.

Nach 5 Stunden wurde das Auftreten und die Schwere der Schäden bei Verwendung von Blindversuchstieren, denen nichts verabreicht wurde, als Kontrolle nach der Verfahrensweise von Carmichael bestimmt.

c) Mit absolutem Äthanol erzeugte Magenschäden

Es wurden 24 Stunden lang nüchtern gehaltene männliche Sprague-Dawley-Retten verwendet. Die Tiere wurden peroral mit 1 ml absolutem Äthanol behandelt.

Die zu untersuchenden Substanzen wurden 1 Stunde vor der Verabreichung des Schädigungsmittels in Lösung in destilliertem Wasser in Dosen von 100 mg/kg Körpergewicht in Volumina von 2 ml/kg Körpergewicht peroral verabreicht.

1 Stunde später wurde die Bestimmung des Auftretens und der Schwere der Schäden bei Verwendung von Blindversuchstieren, denen nichts verabreicht wurde, als Kontrolle nach einer willkürlichen Skala von 0 bis 5 durchgeführt.

Die Ergebnisse dieser Versuche sind in der folgenden Tabelle 1 zusammengestellt.

**Tabelle I**

Geschwürhemmende Wirkung an Ratten

| Erzeugung der Schäden durch beziehungsweise > mit | Behandlung mit (außer in den Blindversuchen perorale Dosis von 100 mg/kg | Zahl der Ratten | Schädigungsindex (Durchschnitt ± statischer Fehler) |
|---|---|---|---|
| Streß | Dinatriumsalz der 3β-[3'-(Carboxypropionyloxy)]-ursa-9(11),12-dien-28-carbonsäure {erfindungsgemärß} | 15 | 1,53 ± 0,53*) |
| " | 3β-[3'-(Carboxypropionyloxy)]-11-oxo-olean-12-en-30-carbonsäure <Carbenoxolon> {Vergleichssubstanz} | 15 | 2,40 ± 0,58*) |
| " | Blind- beziehungsweise Kontrollversuch (keine Behandlung) | 14 | 5,07 ± 1,47 |
| Acetylsalicylsäure | Dinatriumsalz der 3β-[3'-(Carboxypropionyloxy)]-ursa-9(11),12-dien-28-carbonsäure {erfindungsgemäß} | 15 | 11,30 ± 2,32*) |
| " | 3β-[3'-(Carboxypropionyloxy)]-11-oxo-olean-12-en-30-carbonsäure <Carbeoxolon> {Vergleichssubstanz} | 15 | 9,60 ± 2,44*) |
| " | Blind- beziehungsweise Kontrollversuch (keine Behandlung) | 15 | 27,50 ± 6,35 |
| Absolutes Äthanol | Dinatriumsalz der 3β-[3'-(Carboxypropionyloxy)]-ursa-9(11),12-dien-28-carbonsäure {erfindungsgemäß} | 8 | 0,62 ± 0,18**) |
| " | 3β-[3'-(Carboxypropionyloxy)]-11-oxo-olean-12-en-30-carbonsäure <Carbenoxolon> {Vergleichssubstanz} | 8 | 0,37 ± 0,18**) |
| " | Blind- beziehungsweise Kontrollversuch (keine Behandlung) | 8 | 2,37 ± 0,49 |

*) $P < 0,05$ im Vergleich zur Blindversuchs- beziehungsweise Kontrollgruppe (Prüfversuch von Dunnett) **) $P < 0,01$ " "

II) Mineralcorticoidwirksamkeit

Die Mineralcorticoidwirksamkeit des erfindungsgemäßen Dinatriumsalzes der 3β-[3'-(Carboxypropionyloxy)]-ursa-9(11),12-dien-28-carbonsäure und der Vergleichssubstanz 3β-[3'-(Carboxypropionyloxy)]-11-oxo-olean-12-en-30-carbonsäure <Carbenoxolon> wurde unter Verwendung von männlichen Sprague-Dawley-Ratten untersucht.

Den 24 Stunden lang nüchtern gehaltenen Tieren wurden peroral 5 ml destilliertes Wasser und 1 Stunde später die zu untersuchende Substanz in den in der folgenden Tabelle II angegebenen Dosen in Lösung in einem Volumen von 5 ml/kg Körpergewicht physiologischer Natriumchloridlösung verabreicht. Dann wurden die Tiere in Stoffwechselkäfige eingebracht und 5 Stunden lang in diesen gehalten. Die im gesammelten Urin bastimmten Parameter sowie die erhaltenen Ergebnisse sind in der folgenden Tabelle II zusammengestellt. Die Natrium- und Kaliumkonzentrationen wurden durch Flammenphotometrie bestimmt.

**Tabelle II**

Mineralcorticoidwirksamkeit (Diurese) an Ratten

| Behandlung: Substanz | perorale Dosis in mg/kg | Harnausscheidung, prozentuale Änderung, bezogen auf die Blindversuchs- beziehungsweise Kontrollgruppe: Volumen | Na+: Konzen-tration/l | Na+: Ausschei-dung in 5 Stunden | K+: Konzen-tration/l | K+: Ausschei-dung in 5 Stunden | Änderung des Verhältnisses von $\frac{Na^+}{K^+}$ °) |
|---|---|---|---|---|---|---|---|
| Dinatriumsalz der 3β-[3'-(Carboxypropionyloxy)]-ursa-9(11),12-dien-28-carbonsäure {Vergleichssubstanz} | 30 | -13 | +30 | +13 | +21 | +3 | +8 |
| | 100 | -22*) | +35*) | +8 | +34 | +4 | +9 |
| 3β-[3'-(Carboxypropionyloxy)]-11-oxo-olean-12-en-30-carbonsäure <Carbenoxolon> {Vergleichssubstanz} | 10 | -12 | -13 | -23 | +37 | +22 | -35*) |
| | 30 | -15 | -13 | -26*) | +57*) | +35*) | -44**) |
| | 100 | -33**) | -33**) | -54**) | +150**) | +66**) | -73**) |

*) $P < 0{,}05$
**) $P < 0{,}01$
°) im Vergleich zur Blindversuchs- beziehungsweise Kontrollgruppe (Prüfversuch von Dunnett)

In den folgenden Tabellen III und IV sind die Toxizität beziehungsweise therapeutischen Indices der geschwürhemmenden Wirkung an Ratten des erfindungsgemäßen Dinatriumsalzes der 3β-[3'-(Carboxypropionyloxy)]-ursa-9(11),12-dien-28-carbonsäure und der Vergleichssubstanz 3β-[3'-(Carboxypropionyloxy)]-11-oxo-olean-12-en-30-carbonsäure <Carbenoxolon> zusammengestellt.

**Tabelle III**

Toxizität an Ratten

| Verbindung | $LD_{50}$-Werte in mg/kg: peroral | $LD_{50}$-Werte in mg/kg: intraperitoneal |
|---|---|---|
| Dinatriumsalz der 3β-[3'-(Carboxypropionyloxy)]-ursa-9(11),12-dien-28-carbonsäure {erfindungsgemäß} | >5000 | 300 |
| 3β-[3'-(Carboxypropionyloxy)]-11-oxo-olean-12-en-30-carbonsäure <Carbenoxolon> {Vergleichssubstanz} | 3500 | 200 |

**Tabelle IV**

Therapeutische Indices der geschwürhemmenden Wirkung an Ratten

| Verbindung | $\frac{\text{Peroraler } LD_{50}\text{-Wert}}{\text{Schädigungsindex}}$ mit Streß | mit Acetylsalicylsäure | mit absolutem Äthanol | $\frac{\text{Intraperitonealer } LD_{50}\text{-Wert}}{\text{Schädigungsindex}}$ mit Streß | mit Acetylsalicylsäure | mit absolutem Äthanol |
|---|---|---|---|---|---|---|
| Dinatriumsalz der 3β-[3'-(Carboxypropionyloxy)]-ursa-9(11),12-dien-28-carbonsäure {erfindungsgemäß} | > 3 268 | > 443 | > 8 065 | 196 | 27 | 484 |
| 3β-[3'-(Carboxypropionyloxy)]-11-oxo-olean-12-en-30-carbonsäure <Carbenoxolon> {Vergleichssubstanz} | 1 458 | 365 | 9 460 | 83 | 21 | 541 |

Aus den obigen Tabellen 1 bis IV geht eindeutig hervor, daß die erfindungsgemäße Verbindung bei etwa gleicher geschwürhemmender Wirksamkeit wie die der Vergleichssubstanz und etwa gleichen bis höheren therapeutischen Indices wie beziehungsweise als die der Vergleichssubstanz im Gegensatz zu dieser praktisch frei von Mineralcorticoidwirksamkeit ist, was einen großen Vorteil darstellt.

Die erfindungsgemäßen Arzneimittel können in Form von Arzneimittelpräparaten zusammen mit 1 oder mehr festen oder flüssigen Träger- und/oder Hilfsstoff(en) vorliegen und so an Menschen verabreicht werden.

Die Erfindung wird an Hand der folgenden Beispiele näher erläutert.

**Beispiel 1**

Dinatriumsalz der 3β-[3'-(Carboxypropionyloxy)]-ursa-9(11),12-dien-28-carbonsäure

Es wurde eine Lösung von 58 g 3β-(Hydroxy)-ursa-9(11),12-dien-28-carbonsäure und 64 g Bernsteinsäureanhydrid in 450 ml Pyridin etwa 18 Stunden lang unter Rückfluß zum Sieden erhitzt, worauf diese Lösung langsam unter Rühren beziehungsweise Schütteln zu 450 ml einer 96 gew.-%-igen Schwefelsäure in 5 l Eis zugegeben wurde. Der so erhaltene Niederschlag wurde unter Vakuum gesammelt, 2-mal mit je 200 ml Wasser gewaschen und bis zur Erreichung der Gewichtskonstanz getrocknet und dann an Siliciumdioxyd chromatographiert (Eluiermittel: Gemisch von Methylenchlorid und Tetrahydrofuran im Volumverhältnis von 95: 5).

So wurden 30 g {42,39 % der Theorie, bezogen auf die eingesetzte 3β-(Hydroxy)-ursa-9(11),12-dien-28-carbonsäure} 3β-[3'-(Carboxypropionyloxy)]-ursa-9(11),12-dien-28-carbonsäure mit einem Schmelzpunkt von 219 bis 223°C erhalten.

Es wurden einer Lösung der erhaltenen 30 g 3β-[3'-(Carboxypropionyloxy)]-ursa-9(11),12-dien-28-carbonsäure in 1 200 ml Tetrahydrofuran unter Rühren beziehungsweise Schütteln 108 ml einer methanolischen n Natriumhydroxydlösung zugetropft. Es wurde noch 10 Minuten gerührt beziehungsweise geschüttelt, worauf der erhaltene Niederschlag unter Vakuum abfiltriert, 2-mal mit je 50 ml Tetrahydrofuran gewaschen und bis zur Erreichung der Gewichtskonstanz getrocknet wurde. So wurden 30,6 g {94,5 % der Theorie, bezogen auf die eingesetzte 3β-[3'-(Carboxypropionyloxy)]-ursa-9(11),12-dien-28-carbonsäure} des Dinatriumsalzes der 3β-[3'-(Carboxypropionyloxy)]-ursa-9(11),12-dien-28-carbonsäure mit einem Schmelzpunkt von 328 bis 331°C (unter Zersetzung) erhalten.

Die als Ausgangssubstanz verwendete 3β-(Hydroxy)-ursa-9(11),12-dien-28-carbonsäure ist wie folgt beschrieben hergestellt worden.

Es wurden 60 g 3β-(Hydroxy)-ursa-12-en-28-carbonsäure und 12 g Natriumacetat mit 480 ml Eisessig und 120 ml Essigsäureanhydrid behandelt und bis zum vollständigen Lösen unter Rückfluß zum Sieden erhitzt. Dann wurde die Lösung noch 2 Stunden lang unter diesen Bedingungen gehalten, worauf ihr 2 l Wasser zugesetzt wurden und noch 2 Stunden gerührt beziehungsweise geschüttelt wurde. Der Niederschlag wurde abfiltriert, 3-mal mit je 100 ml Wasser gewaschen und dann unter Vakuum bis zur Erreichung der Gewichtskonstanz getrocknet. Es wurden 64,5 g 3β-(Acetoxy)-ursa-12-en-28-carbonsäure mit einem Schmelzpunkt von 158 bis 180°C erhalten.

Es wurden 64 g der erhaltenen 3β-(Acetoxy)-ursa-12-en-28-carbonsäure und 30 g N-(Brom)-succinimid in 1,5 l Tetrachlorkohlenstoff $3\frac{1}{2}$ Stunden lang unter Rückfluß und unter Rühren beziehungsweise Schütteln zum Sieden erhitzt. Dann wurde filtriert und der erhaltene Rückstand wurde 2-mal mit je 20 ml Methylenchlorid gewaschen. Daraufhin wurde das mit den Waschflüssigkeiten vereinigte Filtrat 3-mal mit je 300 ml einer gesättigten Natriumpyrosulfitlösung (Natriummetabisulfitlösung) und 3-mal mit je 350 ml einer gesättigten Ammoniumsulfatlösung gewaschen. Es wurde über wasserfreiem Magnesiumsulfat wasserfrei gemacht und unter Vakuum eingedampft und der erhaltene Rückstand wurde in 450 ml 96 %-igen Äthylalkohol aufgenommen, worauf das Ganze zu einer Lösung von 450 ml einer 30 gew.-%-igen Natriumhydroxydlösung zugegeben und die Mischung 2 Stunden lang unter Rückfluß zum Sieden erhitzt wurde. Etwa 350 ml Äthylalkohol wurden unter Vakuum abdestilliert, worauf mit einer 20 gew.-%-igen Schwefelsäure gegenüber Kongorot angesäuert wurde. Die Reaktionsmischung wurde unter Vakuum filtriert und der erhaltene Rückstand wurde 2-mal mit je 100 ml Wasser gewaschen und unter Vakuum bis zur Erreichung der Gewichtskonstanz getrocknet. Es wurden 58 g {98,44 % der Theorie, bezogen auf die eingesetzte 3β-(Acetoxy)-ursa-12-en-28-carbonsäure} 3β-(Hydroxy)-ursa-9(11),12-dien-28-carbonsäure mit einem Schmelzpunkt von 200 bis 230°C erhalten.

**Beispiel 2**

Dinatriumsalz der 3β-[3'-(Carboxypropionyloxy)]-ursa-9(11),12-dien-28-carbonsäure

Es wurde eine Lösung von 58 g wie im Beispiel 1 beschrieben erhaltener 3β-(Hydroxy)-ursa-9(11),12-dien-28-carbonsäure, 126 g Bernsteinsäureanhydrid und 10 g 4-Di-methylaminopyridin in 1 800 ml Pyridin 48 Stunden lang bei Raumtemperatur gerührt beziehungsweise geschüttelt und dann 3 Stunden lang auf 60°C erhitzt. Die Reaktionsmischung wurde in 9 l einer 20 gew.-%-igen Schwefelsäure eingegossen, der erhaltene Niederschlag

wurde unter Vakuum gesammelt und an Siliciumdioxyd mit Athylacetat als Eluiermittel chromatographiert. So wurden 48,5 g {67,82 % der Theorie, bezogen auf die eingesetzte 3β-(Acetoxy)-ursa-12-en-28-carbonsäure} 3β-[3'-(Carboxypropionyloxy)]-ursa-9(11),12-dien-28-carbonsäure mit einem Schmelzpunkt von 218 bis 222°C erhalten.

Es wurden einer Lösung der erhaltenen 48,5 g 3β-[3'-(Carboxypropionyloxy)]-ursa-9(11),12-dien-28-carbonsäure in einem Gemisch von 600 ml Tetrahydrofuran und 2 400 ml Aceton bei Raumtemperatur unter Rühren beziehungsweise Schütteln 174,6 ml einer methanolischen n Natriumhydroxydlösung zugetropft. Der Niederschlag wurde unter Vakuum filtriert, in Aceton zerrieben und erneut unter Vakuum filtriert. So wurden 47,3 g {90,35 % der Theorie, bezogen auf die eingesetzte 3β-(Hydroxy)-ursa-9(11),12-dien-28-carbonsäure} des Dinatriumsalzes der 3β-[3'-(Carboxypropionyloxy)]-ursa-9(11),12-dien-28-carbonsäure erhalten. Dieses war mit dem Produkt des Beispieles 1 identisch.

**Patentansprüche** für die Vertragsstaaten: BE, CH/LI, DE, FR, GB, IT, LU, NL, SE.

1. 3β-[3'-(Carboxypropionyloxy)]-ursa-9(11),12-dien-28-carbonsäure der Formel

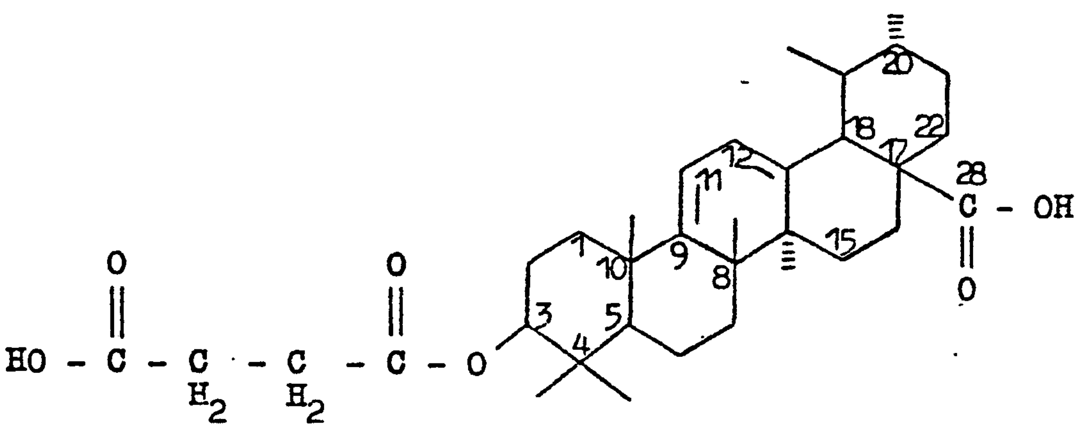

sowie ihre Salze.

2. Dinatriumsalz der 3β-[3'-(Carboxypropionyloxy)]-ursa-9(11),12-dien-28-carbonsäure.

3. Verfahren zur Herstellung der Verbindungen nach Anspruch 1 oder 2, dadurch gekennzeichnet, daß man in an sich bekannter Weise 3β-[Hydroxy]-ursa-9(11),12-dien-28-carbonsäure succinyliert, worauf man in an sich bekannter Weise gegebenenfalls die erhaltene 3β-[3'-(Carboxypropionyloxy)]-ursa-9(11),12-dien-28-carbonsäure in Salze überführt beziehungsweise gegebenenfalls die erhaltenen Salze der 3β-[3'-(Carboxypropionyloxy)]-ursa-9(11),12-dien-28-carbonsäure in die freie 3β-[3'-(Carboxypropionyloxy)]-ursa-9(11),12-dien-28-carbonsäure oder in andere Salze überführt.

4. Arzneimittel, gekennzeichnet durch einen Gehalt an 1 oder mehr Verbindungen nach Anspruch 1 oder 2 als Wirkstoff(en), gegebenenfalls zusammen mit 1 oder mehr üblichen pharmazeutischen Konfektionierungsmittel(n).

**Patentansprüche** für den Vertragsstaat: AT.

1. Verfahren zur Herstellung von 3β-[3'-(Carboxypropionyloxy)]-ursa-9(11),12-dien-28-carbonsäure der Formel

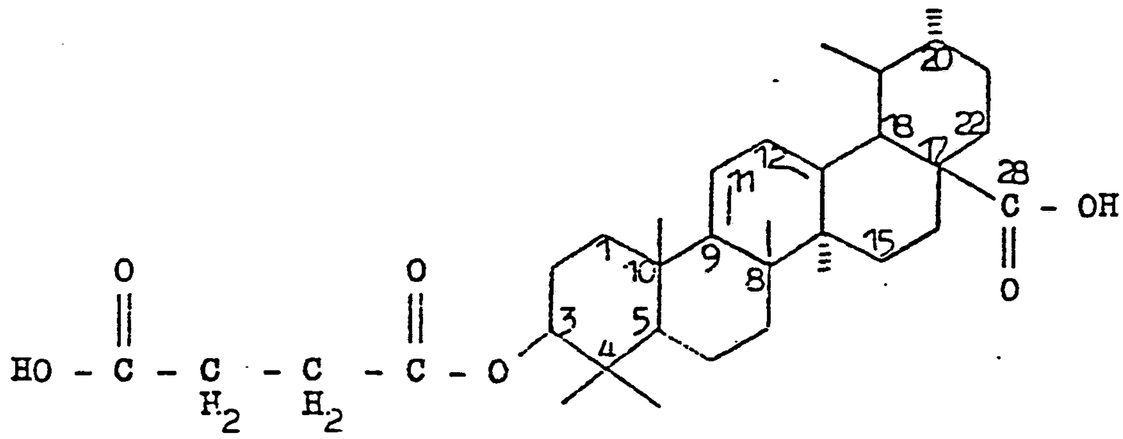

sowie gegebenenfalls ihrer Salze, dadurch gekennzeichnet, daß man in an sich bekannter Weise 3β-[Hydroxy]-ursa-9(11),12-dien-28-carbonsäure succinyliert, worauf man in an sich bekannter Weise gegebenenfalls die erhaltene 3β-[3'-(Carboxypropionyloxy)]-ursa-9(11),12-dien-28-carbonsäure in Salze überführt bzw. gegebenenfalls die erhaltenen Salze der 3β-[3'-(Carboxypropionyloxy)]-ursa-9(11),12-dien-28-carbonsäure in die freie 3β-[3'-(Carboxypropionyloxy)]-ursa-9(11),12-dien-28-carbonsäure oder in andere Salze überführt.

2. Verfahren nach Anspruch 1, dadurch gekennzeichnet, daß das Dinatriumsalz der 3β-[3'-(Carboxypropionyloxy)]-ursa-9(11),12-dien-28-carbonsäure hergestellt wird.

**Claims** for the Contracting States: BE, CH, DE, FR, GB, IT, LI, LU, NL, SE.

1. 3β-[3'-(Carboxypropionyloxy)]-ursa-9(11),12-diene-28-carboxylic acid of the formula

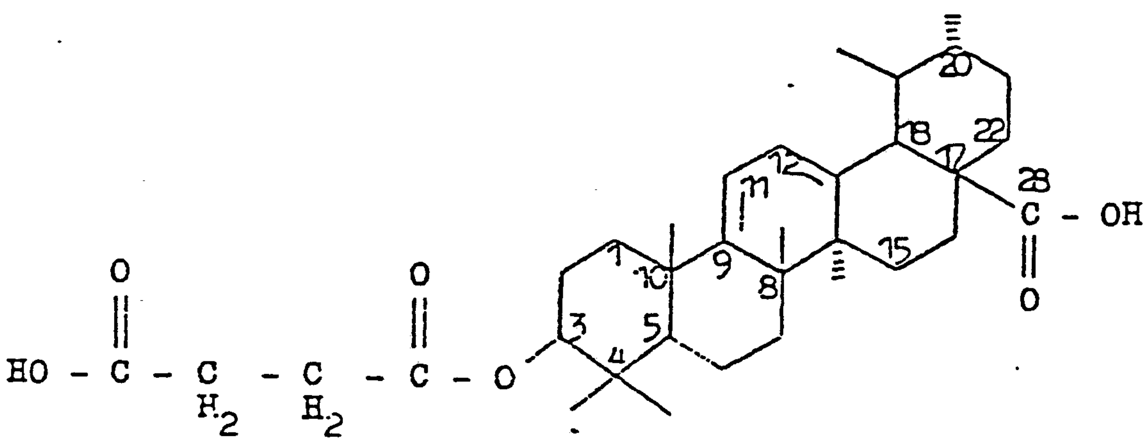

and the salts thereof.

2. Disodium salt of 3β-[3'-(carboxypropionyloxy)]-ursa-9(11),12-diene-28-carboxylic acid.

3. A process for preparing the compounds according to claim 1 or 2, characterized by the fact that in a manner known per se 3β-[hydroxy]-ursa-9(11),12-diene-28-carboxylic is succinylated, and subsequently in a manner known per se optionally the obtained 3β-[3'-(carboxypropionyloxy)]-ursa-9(11),12-diene-28-carboxylic acid is converted into salts and optionally the obtained salts of the 3β-[3'-(carboxypropionyloxy)]-ursa-9(11),12-diene-28-carboxylic acid are converted into the free 3β-[3'-(carboxypropionyloxy)]-ursa-9(11),12-diene-28-carboxylic acid or in different salts, respectively.

4. Pharmaceutical composition, characterized by a content of 1 or more compound(s) according to claim 1 or 2 as active ingredient(s), optionally together with 1 or more conventional pharmaceutical processing aid(s).

**Claims** for the Contracting State AT.

1. A process for preparing 3β-[3'-(carboxypropionyloxy)]-ursa-9(11),12-diene-28-carboxylic acid of the formula

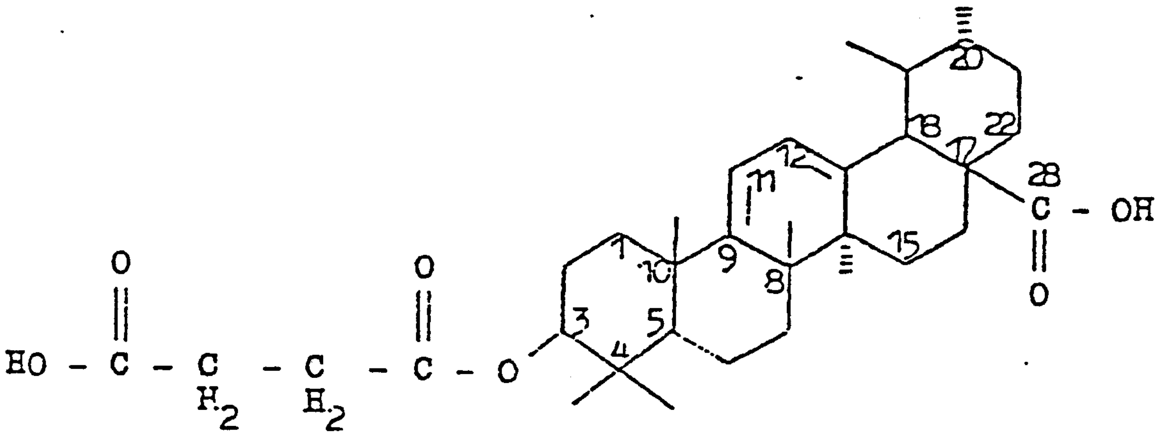

and optionally the salts thereof, characterized by the fact that in a manner known per se 3β-[hydroxy]-ursa-9(11),12-diene-28-carboxylic acid is succinylated, and subsequently in a manner known per se optionally the obtained 3β-[3'-(carboxypropionyloxy)]-ursa-9(11),12-diene-28-carboxylic acid is converted into salts and optionally the obtained salts of the 3β-[3'-(carboxypropionyloxy)]-ursa-9(11),12-diene-28-carboxylic acid are converted into the free 3β-[3'-(carboxypropionyloxy)]-ursa-9(11),12-diene-28-carboxylic acid or in different salts, respectively.

2. A process according to claim 1, characterized by the fact that the disodium salt of 3β-[3'-(carboxypropionyloxy)]-ursa-9(11),12-diene-28-carboxylic acid is prepared.

**Revendications** pour les Etats contractants BE, CH, DE, FR, GB, IT, LI, LU, NL, SE.

1. Acide 3β-[3'-(Carboxypropionyloxy)]-ursa-9(11),12-dien-28-oique de la formule

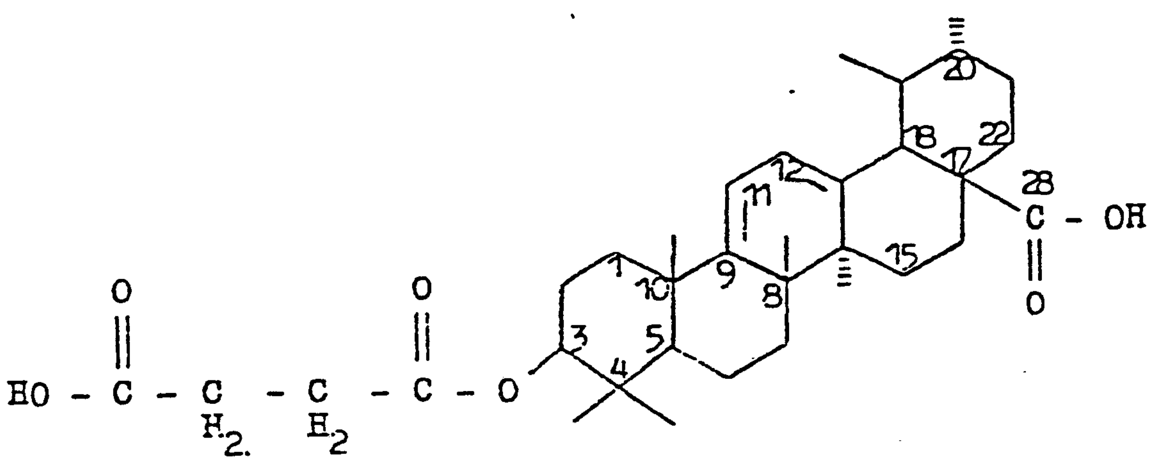

ainsi que leurs sels.

2. Sel disodique de l'acide 3β-[3'-(carboxypropionyloxy)]-ursa-9(11),12-dien-28-oique.

3. Procédé pour la preparation des composés selon la revendication 1 ou 2, caractérisé en ce que on

succinyle en une mode connue en soi l'acide 3β-[hydroxy]-ursa-9(11),12-dien-28-oique après quoi en une mode connue en soi eventuellement on transforme l'acide 3-β-[3'-(carboxypropionyloxy)]-ursa-9(11),12-dien-28-oique obtenue en sels ou eventuellement on transforme les sels de l'acide 3β-[3'-(carboxypropionyloxy)]-ursa-9(11),12-dien-28-oique obtenues en l'acide 3β-[3'-(carboxypropionyloxy)]-ursa-9(11),12-dien-28-oique libre ou en autres sels.

4. Médicaments, caractérisés en un teneur en 1 ou plus composés selon la revendication 1 ou 2 commes principe(s) active(s), eventuellement ensemble avec 1 ou plus agent(s) confectionnant(s) pharmaceutique(s) usuel(s).

**Revendications** pour l'Etat contractant AT

1. Procédé pour la preparation de l'acide 3β-[3'-(carboxypropionyloxy)]-ursa-9(11),12-dien-28-oique de la formule

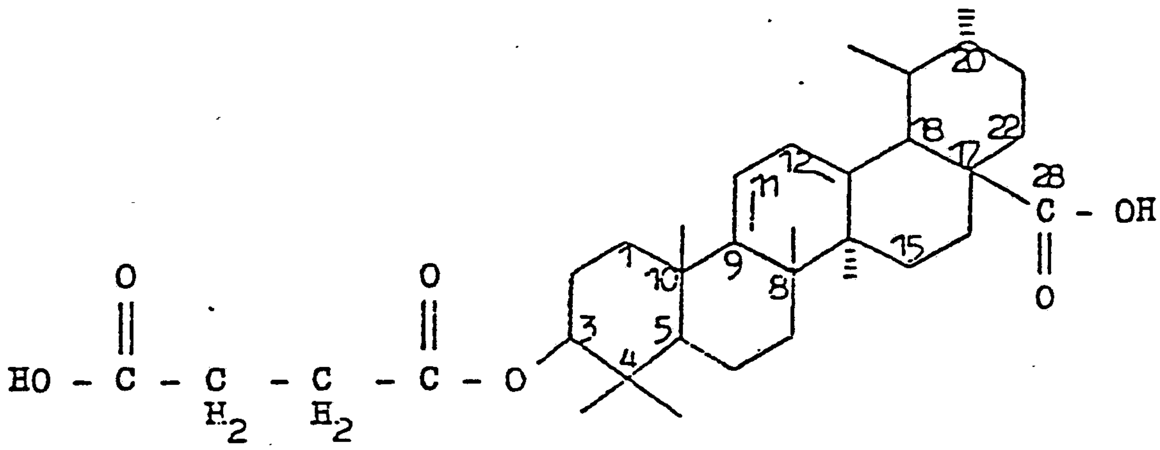

ainsi que eventuellement de leurs sels, caractérisé en ce que on succinyle en une mode connue en soi l'acide 3β-[hydroxy]-ursa-9(11),12-dien-28-oique après quoi en une mode connue en soi eventuellement on transforme l'acide 3β-[3'-(carboxypropionyloxy)]-ursa-9(11),12-dien-28-oique obtenue en sels ou eventuellement on transforme les sels de l'acide 3β-[3'-(carboxypropionyloxy)]-ursa-9(11),12-dien-28-oique obtenues en l'acide 3β-[3'-(carboxypropionyloxy)]-ursa-9(11),12-dien-28-oique libre ou en autres sels.

2. Procédé selon la revendication 1, caractérisé en ce que on prépare le sel disodique de l'acide 3β-[3'-(carboxy-propionyloxy)]-ursa-9(11),12-dien-28-oique.